# FASCICULE DE BREVET EUROPEEN

(11) **EP 1 245 552 B1**
(45) Date de publication et mention de la délivrance du brevet: **13.06.2007**
(21) Numéro de dépôt: 02290655.6
(22) Date de dépôt: 15.03.2002
(51) Int. Cl.: C07C 15/08, C07C 7/13

(54) **Procédé de coproduction de para-xylène et de méta-xylène comprenant deux étapes de séparation**
Verfahren zur gleichzeitigen Produktion von para-Xylol und von meta-Xylol, das zwei Trennungsstufen umfasst
Process for the coproduction of para-xylene and of meta-xylene comprising two separation steps

(30) Priorité: 29.03.2001 FR 0104296
(43) Date de publication de la demande: 02.10.2002
(73) Titulaire: Institut Français du Pétrole, 92852 Rueil-Malmaison Cedex (FR)
(72) Inventeur: Leflaive, Philibert, Les Jardins de Bures - Bat.A, 91440 Bures sur Yvette (FR); Methivier, Alain, 78160 Marly Le Roi (FR); Hotier, Gérard, 92500 Rueil Malmaison (FR)

(56) Documents cités:
- FR-A- 2 782 714
- FR-A- 2 808 270

## Description

L'invention concerne un procédé de coproduction de paraxylène et de métaxylène à partir d'une charge d'hydrocarbures les contenant, le procédé comprenant deux étapes de séparation.

La production de paraxylène de haute pureté par séparation par adsorption est bien connue de l'art antérieur. Ce marché s'est largement développé, ses débouchés sont les productions d'acide téréphtalique, d'anhydride phtalique et de résines polyéthylène téréphtalate. Le marché du métaxylène est au contraire encore restreint, son débouché étant l'acide isophtalique. L'art antérieur connaît cependant des procédés de production de métaxylène de haute pureté par exemple US 4 326 092, US 5 382 747 et US 5 900 523. On s'est récemment aperçu que l'adjonction de faibles quantité de polyéthylène isophtalate au polyéthylène téréphtalate améliorait les propriétés de ce dernier. Il devient donc intéressant de coproduire du paraxylène et du métaxylène dans le même complexe de production d'aromatiques, à condition de satisfaire les exigences du marché : la quantité de paraxylène doit être plus importante que celle de métaxylène : typiquement 2 à 40 fois plus grande, le paraxylène doit être très pur, typiquement au moins 99,6 % et le métaxylène doit être d'une pureté raisonnable, typiquement au moins 99,0%.

L'art antérieur décrit également des procédés de coproduction de para- et de métaxylène par exemple US 4 368 347 utilise un procédé en phase vapeur avec recyclage des fractions intermédiaires : outre la complication liée aux recyclages de fractions intermédiaires, ce document ne décrit pas et ne suggère pas comment il est possible de mettre en oeuvre de manière pratique un tel procédé fonctionnant à pression comprise entre 1 et 2 bar et à une température de 150°C à 200°C avec une charge dont le point de bulle est 145°C et avec des lits fixes présentant des pertes de charges d'au moins 0,1 bar et probablement plus pour fonctionner de manière économique. Le brevet FR 2 651 148 utilise deux solvants différents pour séparer la coupe C8-aromatiques en trois effluents, ce qui limite fortement sa portée puisque les distillations consécutives à l'unité de séparation en lit mobile simulé sont multipliées. Le brevet WO 93/22022 décrit différents cas de séparations de charges de trois constituants en trois effluents, cependant la technologie mise en oeuvre qui implique à la fois des très hautes pressions, une régulation de pression et une régulation de débit dans chacune des trois ou quatre zones du procédé et des lits séparés chacun dans une colonne ne se justifie économiquement que pour des produits de très haute valeur ajoutée.

Le brevet US 4 306 107 décrit un procédé en lit mobile simulé en phase liquide où le métaxylène est prélevé sous forme d'extrait, le paraxylène, l'orthoxylène et une fraction de l'éthylbenzène sont prélevés en tant que raffinat intermédiaire, enfin l'éthylbenzène est prélevé comme raffinat. Ce procédé permettant la coproduction de métaxylène et d'éthylbenzène ne permet naturellement pas de coproduire une majorité de paraxylène et un courant annexe de métaxylène.
Le brevet US 4 313 015 décrit un procédé de coproduction en continu de paraxylène et de métaxylène à partir d'une charge d'hydrocarbures en lit mobile simulé en phase liquide comprenant trois prélèvements. L'extrait est constitué de paraxylène trop impur (99,44%) pour être commercialisé aux normes actuelles (standard actuel = 99,7 mini) et avec un rendement de 97,5%, le raffinat intermédiaire est constitué d'éthylbenzène, d'ortho et de métaxylène et d'un peu de paraxylène, enfin le raffinat est constitué principalement d'un mélange d'orthoxylène et de métaxylène. Du métaxylène pur est alors obtenu par distillation du raffinat.
Un procédé de coproduction continue de paraxylène et de métaxylène à partir d'une charge d'hydrocarbures en lit mobile simulé en phase liquide comprenant trois prélèvements est également décrit dans le brevet FR 2 782 714. La colonne chromatographique décrite contient au moins vingt-cinq lits répartis sur cinq zones. Au moins cinq lits doivent se situer dans la zone 3B comprise entre le point de soutirage d'un raffinat intermédiaire contenant du métaxylène, de l'orthoxylène, de l'éthylbenzène, du solvant et du paraxylène et le point de soutirage d'un raffinat contenant du métaxylène, de l'orthoxylène et du solvant. Du métaxylène d'une pureté supérieure à 99% est alors obtenu par distillation du raffinat. Outre le grand nombre de lits nécessaires à la mise en oeuvre du procédé (30 par exemple), la charge d'hydrocarbures présente une teneur en éthylbenzène inférieure à 5%, ce qui est contraignant.
La demanderesse a déposé une demande de brevet FR 2 808 270 qui décrit un procédé de coproduction en lit mobile simulé de paraxylène et de métaxylène dans une colonne chromatographique comprenant trois prélèvements à partir d'une charge non limitée en éthylbenzène où un extrait contenant du paraxylène est soutiré de manière continue, le premier raffinat est soutiré de manière continue ou discontinue et où le second raffinat comprenant de l'orthoxylène et du métaxylène est soutiré de manière discontinue, le procédé étant en outre caractérisé en ce qu'on distille le second raffinat de façon à récupérer de l'orthoxylène et du métaxylène à au moins 99% de pureté.
Le document US 5 510 562 décrit également un procédé de séparation des aromatiques en C8 où le mélange d'orthoxylène, de métaxylène, de paraxylène, et d'éthylbenzène est d'abord divisé en deux flux contenant respectivement le paraxylène et l'éthylbenzène, et le métaxylène et l'orthoxylène. Le paraxylène est ensuite séparé de l'ethylbenzène par une distillation suivie d'une cristallisation et le métaxylène est séparé de l'orthoxylène par distillation.
Dans tous les procédés décrits dans les brevets US 4 313 015, FR 2 782 714 et US 5 510 562 ainsi que dans la demande de brevet FR 2 808 270, le métaxylène est séparé de l'orthoxylène par distillation. Or, les points d'ébullition de ces deux composés sont très proches (i.e. respectivement 139,12 et 144,41) ce qui rend la séparation de ces deux composés par distillation très difficile et nécessite une colonne importante avec au moins environ 150 à 200 plateaux. De plus, si le flux de mélange de métaxylène et d'orthoxylène que l'on cherche à séparer contient du paraxylène et de l'éthylbenzène en impureté, ces impuretés vont se concentrer dans le métaxylène rendant difficile l'obtention d'une pureté supérieure à 99,0%.

Le document de l'art antérieur qui se rapproche le plus de l'invention est le brevet US 5,900,523. Ce document décrit dans l'exemple E un procédé pour la production des xylènes où une première zone de séparation par adsorption parasélective produit un extrait enrichi en paraxylène et un raffinat qui comporte au moins la majorité de l'ortho- et du métaxylène présents dans le flux d'alimentation et qui contient plus de 10 pour cent d'orthoxylène. L'extrait est distillé pour récupérer du paraxylène de haute pureté. Le raffinat de la première zone de séparation est ensuite introduit dans une seconde zone de séparation par adsorption métasélective où l'adsorbant est une zéolithe Y de ratio molaire SiO₂/Al₂O₃ compris entre 4,0 et 6,0 échangée au sodium et ayant une teneur en eau équivalente à un LOI à 500°C d'environ 1,5 à environ 2,5 % poids et où la séparation est conduite en phase liquide à une température comprise entre 100°C et 150°C. La seconde zone de séparation par adsorption métasélective produit un extrait enrichi en métaxylène et un raffinat qui comporte les composés non-adsorbés du premier raffinat, notamment l'orthoxylène. Du métaxylène de haute pureté est récupéré à partir de l'extrait. Il apparaît clairement dans cette description que les deux adsorptions sont placées en série sans qu'une quelconque synergie puisse être trouvée pour la coproduction des deux isomères.

L'objet de l'invention est la coproduction de paraxylène et de métaxylène commercialisables à partir d'une charge d'hydrocarbures. Un second objet de l'invention est d'obtenir du paraxylène à une pureté d'au moins 99,6 %, (avec un rendement minimum de 98 %) et du métaxylène de pureté au moins égale à 99 % après distillation. Un troisième objet de l'invention est de produire du paraxylène et du métaxylène avec deux unités de séparation où la taille de la seconde unité est réduite.

De manière plus précise, l'invention concerne un procédé de coproduction de paraxylène et de métaxylène à partir d'une charge d'hydrocarbures qui les comprend, le procédé comprenant une première étape de séparation de la charge en lit mobile simulé dans au moins une première colonne (6) chromatographique contenant une pluralité de lits d'au moins un adsorbant interconnectés en boucle, ladite colonne comprenant une injection de la charge (1), un soutirage d'un premier raffinat (4), un soutirage d'un second raffinat (5) comprenant du désorbant, et un mélange contenant du métaxylène et de l'orthoxylène sensiblement exempt d'éthylbenzène et de paraxylène, une injection de désorbant (2) et un soutirage d'un extrait délivrant du paraxylène de très haute pureté, le procédé comportant le décalage périodique simultané des positions d'injections de charge et de désorbant et de la position de soutirage de l'extrait, d'un lit dans le sens de l'écoulement d'un flux principal circulant dans ladite première colonne (6), le procédé étant caractérisé en ce qu'on distille le second raffinat pour en éliminer le désorbant, on récupère le mélange (12) contenant du métaxylène et de l'orthoxylène, on réalise une deuxième étape de séparation d'une partie au moins du mélange d'orthoxylène et de métaxylène dans au moins une deuxième colonne (17) chromatographique contenant au moins un adsorbant et comprenant au moins une injection du mélange (12), une injection d'un désorbant (16), un soutirage d'un extrait (18) contenant du désorbant et enrichi en un composé le plus adsorbé sur l'adsorbant et un soutirage d'un raffinat (19) contenant du désorbant et enrichi en un composé le moins adsorbé sur l'adsorbant, le procédé étant en outre caractérisé en ce qu'on distille l'extrait contenant le métaxylène ou le raffinat contenant le métaxylène, pour en éliminer le désorbant et récupérer du métaxylène avec une pureté supérieure à 99%.

La deuxième étape de séparation peut être réalisée en batch. Elle peut aussi être réalisée en continu selon la technique du lit mobile simulé, de préférence avec un lit mobile simulé à contre courant. Pour ce faire, on décale périodiquement et simultanément les positions des injections du mélange et du désorbant et la position des soutirages de l'extrait et du raffinat relatifs à la deuxième colonne chromatographique d'un lit dans le sens d'écoulement d'un flux principal circulant dans ladite deuxième colonne.

Les avantages du procédé selon l'invention par rapport à celui de l'art antérieur sont les suivants:
- Le métaxylène n'est pas séparé de l'orthoxylène par distillation, distillation difficile et coûteuse.
- Pour une production donnée et à isopureté du métaxylène, les dimensions de colonnes de la seconde étape d'adsorption sont réduites par rapport à celles nécessitées par l'art antérieur, par exemple 10% à 20% plus petites.
- On peut produire du paraxylène et du méthaxylène sans contrainte sévère sur le nombre de lits d'adsorbant et sur la teneur en éthylbenzène de la charge.

Lors de la première étape de séparation en lit mobile simulé, le premier et le second raffinat peuvent être soutirés de manière continue ou discontinue. En soutirant le second raffinat de préférence de manière continue, on peut l'injecter en continu dans l'étape de distillation, sans réservoir tampon intermédiaire.
Selon une caractéristique du procédé, l'adsorbant utilisé dans la première étape de séparation peut comprendre une zéolithe X échangée au barium ou une zéolithe Y échangée au potassium ou une zéolithe Y échangée au barium et au potassium.
Le désorbant préféré est le paradiéthylbenzène, cependant d'autres désorbants tels que le toluène, le paradifluorobenzène ou des diéthylbenzènes en mélange peuvent également convenir. On préconise de préférence le paradiéthylbenzène pour sa facilité à le récupérer par distillation et pour sa forte affinité pour l'adsorbant.

Selon une autre caractéristique du procédé, on peut utiliser un adsorbant dans la deuxième étape de séparation des composés ortho- et métaxylène du second raffinat qui est métasélectif. Dans ce cas, l'extrait soutiré contient du désorbant et du métaxylène sensiblement pur, composé le plus adsorbé. Mais on peut aussi utiliser un adsorbant dans lequel le raffinat délivre le métaxylène sensiblement pur et dans lequel l'extrait délivre l'orthoxylène, en solution dans le désorbant avec les impuretés restantes.
Le désorbant préféré dans la deuxième étape de séparation est le toluène, cependant d'autres désorbants tels que l'indane, le 1, 2, 4 Triméthylbenzène, le para méthyl éthylbenzène ou le cumène, purs ou en mélange peuvent également convenir.
L'adsorbant de la deuxième étape de séparation peut comprendre au moins une zéolithe choisie dans le groupe consistant essentiellement en une zéolithe X échangée au calcium, une zéolithe X échangée au césium, une zéolithe Y échangée au sodium ou une zéolithe Y échangée au sodium et au lithium. De préférence on préconise l'utilisation d'une zéolithe Y ne contenant sensiblement que du sodium. Des exemples de zéolithes métasélectives contenant du sodium sont décrits dans les brevets US 4326092, US 5382747, US 5900523 et EP.A.712821.

Selon une autre caractéristique de l'invention, le rapport volumique de désorbant sur charge dans la première étape de séparation peut être compris entre 0,5 et 2,5 , de préférence entre 1 et 2.

Selon une autre caractéristique de l'invention, on peut opérer chacune des étapes du procédé à une température généralement comprise entre 20°C et 250°C, de préférence entre 90°C et 210°C et plus particulièrement entre 160°C et 200°C et sous une pression comprise entre la pression atmosphérique et 20 bar (1 bar = 0,1 MPa)

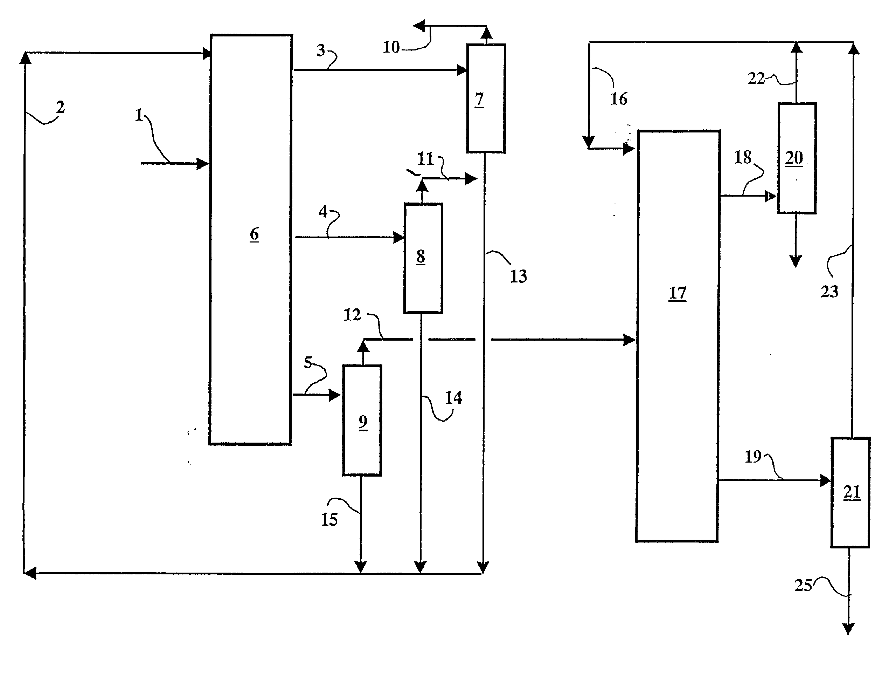

L'invention sera mieux comprise au vu de la figure qui illustre la coproduction de paraxylène et de métaxylène en lit mobile simulé et à contre-courant.
Une charge de xylènes comprenant du métaxylène, de l'orthoxylène, de l'éthylbenzène et du paraxylène est introduite en continu par une ligne (1) dans au moins une colonne (6) chromatographique à au moins cinq zones contenant une pluralité de lits d'un adsorbant comprenant une zéolithe, une zéolithe X échangée au baryum par exemple et fonctionnant en phase liquide en lit mobile simulé et à contre-courant selon le brevet US 4313015 et le brevet déjà cité de la demanderesse. Un premier raffinat R1 est soutiré en continu par une ligne (4) en un point situé en aval du point d'introduction de la charge, tandis qu'un deuxième raffinat R2 contenant du métaxylène et de l'orthoxylène est soutiré en continu par une ligne (5) en aval du premier raffinat par rapport au sens d'écoulement des fluides dans la colonne (spécifiquement de bas en haut). Un désorbant, le paradiéthylbenzène est injecté en continu par une ligne (2) en un point de la colonne situé en amont du point d'injection de la charge alors qu'un extrait contenant du désorbant et du paraxylène sensiblement pur est soutiré en continu par une ligne (3) en un point situé en aval du point d'injection du désorbant. Cet extrait est distillé dans une colonne de distillation (7), de laquelle on soutire en tête le paraxylène sensiblement pur (supérieur à 99,7%) par une ligne (10) et en fond, par une ligne (13) le désorbant qui peut être recyclé.
Le premier raffinat est introduit dans une colonne de distillation (8) de laquelle on soutire en fond par une ligne (14) le désorbant qui peut être recyclé et en tête, un mélange contenant des xylènes et de l'éthylbenzène par une ligne (11). Ce mélange peut être envoyé vers une unité d'isomérisation.

Le deuxième raffinat est introduit dans une colonne de distillation (9) de laquelle on soutire en fond par une ligne (15) le désorbant qui peut être recyclé et en tête, un mélange contenant essentiellement du métaxylène et de l'orthoxylène et sensiblement exempt de paraxylène et d'éthylbenzène par une ligne (12). Cette ligne (12) est connectée à l'entrée d'au moins une deuxième colonne chromatographique (17) comprenant une pluralité de lits d'un adsorbant zéolithique, une zéolithe NaY par exemple, et qui est opérée en phase liquide en lit mobile simulé à contre-courant, par exemple selon le brevet US 4326092 ou US 5382747 avec une zéolithe Y contenant du sodium.
Un désorbant, du toluène par exemple est introduit en continu par une ligne (16) dans la colonne chromatographique (17) en un point situé en amont du point d'introduction de la charge tandis qu'un extrait contenant du métaxylène sensiblement pur et du désorbant est soutiré en continu par une ligne (18) en aval du point d'introduction du désorbant et en amont du point d'introduction de la charge. La ligne (18) d'extrait est connectée à l'entrée d'une colonne de distillation (20) de laquelle on soutire de manière classique le désorbant par une ligne (22) en tête, tandis qu'en fond de colonne on récupère le métaxylène avec une pureté par exemple supérieure à 99%, par une ligne (24).
En aval du point d'injection de ladite charge dans le sens de l'écoulement du fluide principal circulant dans la colonne, on soutire en continu par une ligne (19) un raffinat contenant de l'orthoxylène, des impuretés et du désorbant que l'on distille dans une colonne (21) de distillation. De l'orthoxylène contenant des impuretés est recueilli en fond de colonne (21) par une ligne (25) pour être éventuellement isomérisé avec celui de la ligne 11 tandis que le désorbant est récupéré en tête de la colonne (21) par une ligne (23).
Les points d'introduction de charge et de désorbant et les points de soutirage d'extrait et de raffinat sont décalés périodiquement et simultanément dans le sens de l'écoulement du fluide circulant dans les colonnes.

L'invention est illustrée par les exemples suivants donnés à titre non limitatif.

### Exemple 1 (comparatif)

La production de paraxylène à partir d'une charge comportant un mélange de xylènes et d'éthylbenzène de composition pondérale suivante :
PX : Paraxylène 22,6%
MX : Métaxylène 49,9%
OX : Orthoxylène 21,9%
EB : Ethylbenzène 5,6%
est réalisée en lit mobile simulé à 4 zones, à contre courant dans deux adsorbeurs cylindriques de 1m² de section et composés de 24 lits contenant une zéolithe X échangée au baryum.

Les conditions opératoires sont les suivantes :
Charge : 9,5 m³.h⁻¹
Solvant : 16,2 m³.h⁻¹ de paradiéthylbenzène
Extrait : 9,6 m³.h⁻¹
Raffinat : 16,1 m³.h⁻¹
Débit de recyclage (en zone 1) : 48,2 m³.h⁻¹
La configuration est de 5 lits, 9 lits, 8 lits et 2 lits respectivement dans les zones 1, 2, 3 et 4.

Le temps de permutation des vannes (ou période) est de 70,8 secondes.

Après distillation du paradiéthylbenzène, l'extrait obtenu délivre du paraxylène à 99,7% de pureté et un rendement de 96,7%.

Les 16,1 m³.h⁻¹ de raffinat sont distillés et on obtient un débit de fluide de 7,4 m³.h⁻¹ dont la composition est la suivante :
PX : Paraxylène 1,0%
MX : Métaxylène 63,8%
OX : Orthoxylène 28,0%
EB : Ethylbenzène 7,2%

Une partie, soit 0,642 m³.h⁻¹ de ce fluide est prélevée et envoyée dans une unité de séparation métasélective. La production de métaxylène est réalisée en lit mobile simulé, à contre courant dans deux adsorbeurs cylindriques de 0,0803 m² de section et composés de 24 lits contenant une zéolithe Y échangée au sodium.
Les conditions opératoires sont les suivantes :
Charge : 0,642 m³.h⁻¹
Solvant : 1,027 m³.h⁻¹ de toluène
Extrait : 0,757 m³.h⁻¹
Raffinat : 0,912 m³.h⁻¹
Débit de recyclage (en zone 1) : 4,045 m³.h⁻¹
La configuration est de 3 lits, 11 lits, 7 lits et 3 lits respectivement dans les zones 1, 2, 3 et 4.

Le temps de permutation des vannes (ou période) est de 90 secondes.

Après distillation du toluène, l'extrait obtenu délivre 0,208 m³.h⁻¹ de métaxylène à 99,03% de pureté. La production de paraxylène est 10 fois supérieure à celle de métaxylène.

### Exemple 2

La production de paraxylène à partir d'une charge comportant un mélange de xylène et d'éthylbenzène de composition pondérale suivante :
PX : Paraxylène 22,6%
MX : Métaxylène 49,9%
OX : Orthoxylène 21,9%
EB : Ethylbenzène 5,6%
est réalisée en lit mobile simulé à cinq zones, à contre courant dans deux adsorbeurs cylindriques de 1m² de section et composés de 24 lits contenant une zéolithe X échangée au baryum. On soutire en continu un raffinat (raffinat 2) et un raffinat intermédiaire.

Les conditions opératoires sont les suivantes :
Charge : 9,5 m³.h⁻¹
Solvant : 16,2 m³.h⁻¹ de paradiéthylbenzène
Extrait : 9,6 m³.h⁻¹
Raffinat intermédiaire: 10,1 m³.h⁻¹
Raffinat 2: 6,0 m³.h⁻¹
Débit de recyclage (en zone 1) : 48,2 m³.h⁻¹
La configuration est de 5 lits, 9 lits, 5 lits, 3 lits et 2 lits respectivement dans les zones 1, 2, 3A, 3B et 4.

Le temps de permutation des vannes (ou période) est de 70,8 secondes.

Après distillation du paradiéthylbenzène, l'extrait obtenu soutiré en continu délivre du paraxylène à 99,7% de pureté et un rendement de 96,7%. On constate que dédoubler le raffinat ne dégrade pas le performance de l'unité pour la production du paraxylène.

Les 6,0 m³.h⁻¹ de raffinat 2 sont distillés et on obtient un débit de fluide de 1,13 m³.h⁻¹ dont la composition est la suivante :
PX : Paraxylène 0,7%
MX : Métaxylène 71,7%
OX : Orthoxylène 27,5%
EB : Ethylbenzène 0,1%

Une partie, soit 0,580 m³.h⁻¹ de ce fluide est prélevée et envoyée en continu dans une unité de séparation métasélective. La production de métaxylène est réalisée en lit mobile simulé, à contre courant dans deux adsorbeurs cylindriques de 0,0725 m² de section et composés de 24 lits contenant une zéolithe Y échangée au sodium.
Les conditions opératoires (débits) optimisés sont les suivantes :
Charge : 0,580 m³.h⁻¹
Solvant : 0,928 m³.h⁻¹ de toluène
Extrait : 0,675 m³.h⁻¹
Raffinat : 0,833 m³.h⁻¹
Débit de recyclage (en zone 1) : 3,930 m³.h⁻¹
La configuration est de 3 lits, 11 lits, 8 lits et 2 lits respectivement dans les zones 1, 2, 3 et 4.

Le temps de permutation des vannes (ou période) est de 82 secondes.

Après distillation du toluène, l'extrait obtenu délivre 0,208 m³.h⁻¹ de métaxylène à 99,04% de pureté soit une production de paraxylène 10 fois supérieure à celle de métaxylène.

Dans cet exemple, on constate que la première unité est opérée avec un nombre de lits limité à 24. De plus les quantités d'adsorbant et de solvant nécessaires dans la seconde unité d'adsorption en lit mobile simulé pour une production de métaxylène identique sont environ 10% inférieures à celles de l'exemple 1.

### Exemple 3

La production de paraxylène à partir d'une charge plus riche en éthylbenzène que celle de l'exemple 2 et comportant un mélange de xylènes et d'éthylbenzène de composition pondérale suivante :
PX : Paraxylène 21,1%
MX : Métaxylène 48,9%
OX : Orthoxylène 21,4%
EB : Ethylbenzène 8,6%
est réalisée en lit mobile simulé, à contre courant dans deux adsorbeurs cylindriques de 1m² de section et composés de 24 lits contenant une zéolithe X échangée au baryum. On soutire en continu un raffinat (raffinat 2) et un raffinat intermédiaire.

Les conditions opératoires sont les suivantes :
Charge : 9,5 m³.h⁻¹
Solvant : 16,2 m³.h⁻¹ de paradiéthylbenzène
Extrait : 9,5 m³.h⁻¹
Raffinat intermédiaire: 12,05 m³.h⁻¹
Raffinat 2: 4,15 m³.h⁻¹
Débit de recyclage (en zone 1) : 48,2 m³.h⁻¹
La configuration est de 5 lits, 9 lits, 5 lits, 3 lits et 2 lits respectivement dans les zones 1, 2, 3A, 3B et 4.

Le temps de permutation des vannes (ou période) est de 70,8 secondes.

Après distillation du paradiéthylbenzène, l'extrait obtenu délivre du paraxylène à 99,7% de pureté et un rendement de 96,0%.

Les 4,15 m³.h⁻¹ de raffinat 2 sont distillés et on obtient un débit de fluide de 0,62 m³.h⁻¹ dont la composition est la suivante :
PX : Paraxylène 1,4%
MX : Métaxylène 70,2%
OX : Orthoxylène 28,2%
EB : Ethylbenzène 0,2%
Une partie, soit 0,595 m³.h⁻¹ de ce fluide est prélevée et envoyée en continu dans une unité de séparation métasélective. La production de métaxylène est réalisée en lit mobile simulé, à contre courant dans deux adsorbeurs cylindriques de 0,0745 m² de section et composés de 24 lits contenant une zéolithe Y échangée au sodium.
Les conditions opératoires (débits) optimisés sont les suivantes :
Charge : 0,595 m³.h⁻¹
Solvant : 0,952 m³.h⁻¹ de toluène
Extrait : 0,684 m³.h⁻¹
Raffinat : 0,863 m³.h⁻¹
Débit de recyclage (en zone 1) : 4,028 m³.h⁻¹
La configuration est de 3 lits, 11 lits, 8 lits et 2 lits respectivement dans les zones 1, 2, 3 et 4.

Le temps de permutation des vannes (ou période) est de 82 secondes.

Après distillation du toluène, l'extrait obtenu délivre 0,208 m³.h⁻¹ de métaxylène à 99,05% de pureté soit une production de paraxylène 10 fois supérieure à celle de métaxylène.

## Revendications

1. - Procédé de coproduction de paraxylène et de métaxylène à partir d'une charge d'hydrocarbures qui les comprend, le procédé comprenant une première étape de séparation de la charge en lit mobile simulé dans au moins une première colonne (6) chromatographique contenant une pluralité de lits d'au moins un adsorbant interconnectés en boucle, ladite colonne comprenant une injection de la charge (1), un soutirage d'un premier raffinat (4), un soutirage d'un second raffinat (5) comprenant du désorbant, et un mélange contenant du métaxylène et de l'orthoxylène sensiblement exempt d'éthylbenzène et de paraxylène, une injection de désorbant (2) et un soutirage d'un extrait (3) délivrant du paraxylène de très haute pureté, le procédé comportant le décalage périodique simultané des positions d'injections de charge et de désorbant et de la position de soutirage de l'extrait, d'un lit dans le sens de l'écoulement d'un flux principal circulant dans ladite première colonne (6), le procédé étant **caractérisé en ce qu'**on distille le second raffinat pour en éliminer le désorbant, on récupère le mélange (12) contenant du métaxylène et de l'orthoxylène, on réalise une deuxième étape de séparation d'une partie au moins du mélange d'orthoxylène et de métaxylène dans au moins une deuxième colonne (17) chromatographique contenant au moins un adsorbant et comprenant au moins une injection du mélange (12), une injection d'un désorbant (16), un soutirage d'un extrait (18) contenant du désorbant et enrichi en un composé le plus adsorbé sur l'adsorbant et un soutirage d'un raffinat (19) contenant du désorbant et enrichi en un composé le moins adsorbé sur l'adsorbant, le procédé étant en outre **caractérisé en ce qu**'on distille l'extrait contenant le métaxylène ou le raffinat contenant le métaxylène, pour en éliminer le désorbant et récupérer du métaxylène avec une pureté supérieure à 99%.

2. - Procédé selon la revendication 1 dans lequel la deuxième étape de séparation est réalisée en lit mobile simulé, de préférence à contre courant simulé.

3. - Procédé selon l'une des revendications 1 et 2 dans lequel l'adsorbant de la deuxième étape de séparation est métasélectif et dans lequel l'extrait contient le métaxylène sensiblement pur.

4. - Procédé selon l'une des revendications 1 à 3, dans lequel on soutire le premier et le second raffinat de manière continue, lors de la première étape de séparation.

5. - Procédé selon l'une des revendications 1 à 4, dans lequel le désorbant de la première étape de séparation est le paradiéthylbenzène.

6. - Procédé selon l'une des revendications 1 à 5, dans lequel l'adsorbant de la première étape de séparation comprend une zéolithe X échangée au barium, une zéolithe Y échangée au potassium ou une zéolithe Y échangée au barium et au potassium.

7. - Procédé selon l'une des revendications 1 à 6, dans lequel le désorbant de la deuxième étape de séparation est le toluène ou l'indane.

8. - Procédé selon l'une des revendications 1 à 7, dans lequel l'adsorbant de la deuxième étape de séparation comprend une zéolithe Y contenant du sodium ou une zéolithe Y contenant du sodium et du lithium.

9. - Procédé selon l'une des revendications 1 à 8, dans lequel le rapport désorbant sur charge dans les première et deuxième étapes de séparation est compris entre 0,5 et 2,5, de préférence entre 1 et 2.

10. - Procédé selon l'une des revendications 1 à 9, dans lequel la température des étapes d'adsorption est comprise entre 20 et 250°C à une pression de 1 bar à 20 bar.

## Claims

1. A process for co-producing paraxylene and metaxylene from a hydrocarbon feed comprising them, the process comprising a first step for separating the feed in a simulated moving bed in at least a first chromatographic column (6) containing a plurality of beds of at least one adsorbent interconnected into a loop, said column comprising a feed injection (1), a take-off for a first raffinate (4), a take-off for a second raffinate (5) comprising a desorbent, and a mixture containing metaxylene and orthoxylene that is substantially free of ethylbenzene and paraxylene, an injection point for desorbent (2) and a take-off for an extract delivering very high purity paraxylene, the process comprising periodic simultaneous shifting of the feed and desorbent injection positions and of the extract take-off position, by one bed in the direction of flow of a principal stream moving in said first column (6), the process being **characterized in that** the second raffinate is distilled to eliminate the desorbent, a mixture (12) containing metaxylene and orthoxylene is recovered, a second step for separating at least a portion of the mixture of orthoxylene and metaxylene is carried out in at least one second chromatographic column (17) containing at least one adsorbent and comprising at least one point for injection of the mixture (12), an injection point for a desorbent (16), a take-off for an extract (18) containing desorbent and enriched in the component that is the most adsorbed on the adsorbent, and a take-off for a raffinate (19) containing desorbent and enriched in the compound that is the least adsorbed on the adsorbent, the process being further **characterized in that** the extract containing metaxylene or the raffinate containing metaxylene is distilled to eliminate desorbent and recover metaxylene with a purity of more than 99%.

2. A process according to claim 1, in which the second separation step is carried out in a simulated moving bed, preferably with a simulated counter-current.

3. A process according to claim 1 or claim 2, in which the adsorbent for the second separation step is metaselective and in which the extract contains substantially pure metaxylene.

4. A process according to any one of claims 1 to 3, in which the first and second raffinate are taken off continuously, during the first separation step.

5. A process according to any one of claims 1 to 4, in which the desorbent for the first separation step is para-diethylbenzene.

6. A process according to any one of claims 1 to 5, in which the adsorbent for the first separation step comprises a barium-exchanged X zeolite, a potassium-exchanged Y zeolite or a barium- and potassium-exchanged Y zeolite.

7. A process according to any one of claims 1 to 6, in which the desorbent for the second separation step is toluene or indane.

8. A process according to any one of claims 1 to 7, in which the adsorbent for the second separation step comprises a Y zeolite containing sodium or a Y zeolite containing sodium and lithium.

9. A process according to any one of claims 1 to 8, in which the ratio of desorbent to feed in the first and second separation steps is in the range 0.5 to 2.5, preferably in the range 1 to 2.

10. A process according to any one of claims 1 to 9, in which the temperature of the adsorption steps is in the range 20°C to 250°C at a pressure of 1 bar to 20 bars.

## Patentansprüche

1. Verfahren zur gemeinsamen Herstellung von Para-Xylol und Meta-Xylol aus einer Kohlenwasserstoffbeschickung, die sie umfasst, wobei das Verfahren einen ersten Schritt der Trennung der Beschickung in einem simulierten Bewegtbett in mindestens einer ersten Chromatographiesäule (6) umfasst, die eine Vielzahl von Betten mit mindestens einem Adsorptionsmittel enthält, die untereinander zu einer Schleife verbunden sind, wobei die Säule eine Einspritzung der Beschickung (1), eine Entnahme eines ersten Raffinats (4), eine Entnahme eines zweiten Raffinats (5), das ein Desorbent umfasst, und eine Mischung, die Meta-Xylol und Ortho-Xylol enthält, das im Wesentlichen frei von Ethylbenzol und Para-xylol ist, eine Einspritzung des Desorbent (2) und eine Entnahme eines Extrakts (3) umfasst, der Para-Xylol von sehr hoher Reinheit liefert, wobei das Verfahren die gleichzeitige periodische Verschiebung der Einspitzpositionen der Beschickung und des Desorbent und der Entnahmeposition des Extrakts durch ein Bett in Abflussrichtung eines Hauptstrom der in der ersten Säule (6) zirkuliert, umfasst, wobei das Verfahren **dadurch gekennzeichnet ist, dass** das zweite Raffinat destilliert wird, um das Desorbent zu entfernen, die Mischung (12) zu gewinnen, die Meta-Xylol und Ortho-Xylol enthält, ein zweiter Schritt der Trennung mindestens eines Teils der Mischung aus Ortho-Xylol und Meta-Xylol in mindestens einer zweiten Chromatographiesäule (17) ausgeführt wird, die mindestens ein Adsorptionsmittel enthält und mindestens eine Einspritzung für die Mischung (12), eine Einspritzung für ein Desorbent (16), eine Entnahmestelle für einen Extrakt (18) umfasst, der Desorbent enthält und mit der Verbindung angereichert ist, die auf dem Adsorptionsmittel am meisten adsorbiert wird, und mit einer Entnahme eines Raffinats (19), das Desorbent enthält und mit einer Verbindung angereichert ist, die auf dem Adsorptionsmittel am wenigsten adsorbiert wird, wobei das Verfahren ferner **dadurch gekennzeichnet ist, dass** der Extrakt, der das Meta-Xylol oder das Raffinat, das das Meta-Xylol enthält, destilliert wird, um daraus das Desorbent zu entfernen, und Meta-Xylol mit einer Reinheit von mehr als 99 % zu gewinnen.

2. Verfahren nach Anspruch 1, wobei der zweite Trennungsschritt in einem simulierten Bewegtbett, vorzugsweise mit einem simulierten Gegenstrom, ausgeführt wird.

3. Verfahren nach einem der Ansprüche 1 und 2, wobei das Adsorptionsmittel des zweiten Trennungsschritts metaselektiv ist, und wobei der Extrakt das im Wesentlichen reine Meta-Xylol enthält.

4. Verfahren nach einem der Ansprüche 1 bis 3, wobei das erste und das zweite Raffinat während des ersten Trennungsschritts kontinuierlich entnommen werden.

5. Verfahren nach einem der Ansprüche 1 bis 4, wobei das Desorbent aus dem ersten Trennungsschritt Paradiethylbenzol ist.

6. Verfahren nach einem der Ansprüche 1 bis 5, wobei das Adsorptionsmittel des ersten Trennungsschritts einen Barium-ausgetauschten Zeolith X, einen Kalium-ausgetauschten Zeolith Y oder einen Barium- und Kalium-ausgetauschten Zeolith Y umfasst.

7. Verfahren nach einem der Ansprüche 1 bis 6, wobei das Desorbent aus dem zweiten Trennungsschritt Toluol oder Indan ist.

8. Verfahren nach einem der Ansprüche 1 bis 7, wobei das Adsorptionsmittel des zweiten Trennungsschritts einen Zeolith Y umfasst, der Natrium enthält, oder einen Zeolith Y, der Natrium und Lithium enthält.

9. Verfahren nach einem der Ansprüche 1 bis 8, wobei das Verhältnis Desorbent zur Beschickung in dem ersten und dem zweiten Trennungsschritt im Bereich zwischen 0,5 und 2,5, bevorzugt zwischen 1 und 2 liegt.

10. Verfahren nach einem der Ansprüche 1 bis 9, wobei die Temperatur der Adsorptionsschritte im Bereich zwischen 20 und 250 °C liegt und bei einem Druck von 1 Bar bis 20 Bar.
